# EUROPEAN PATENT SPECIFICATION

(11) **EP 2 295 971 B1**
(45) Date of publication and mention of the grant of the patent: **07.09.2016**
(21) Application number: 10011093.1
(22) Date of filing: 11.03.2002
(51) Int. Cl.: G01N 33/543, A61K 47/48, G01N 33/531, G01N 33/548, G01N 21/00

(54) **Conjugate probes and optical detection of analytes**
Konjugatsonden und optische Erkennung von Analyten
Sondes de conjugué et détection optique d'analytes

(30) Priority: 09.03.2001 US 274177 P
(43) Date of publication of application: 16.03.2011
(62) Divisional of application: 02725114.9
(73) Proprietor: TrovaGene, Inc., San Diego, CA 92121 (US)
(72) Inventor: Liu, Zhiping, Mountain View, CA 94040 (US); Li, Zheng, Hayward, CA 94544 (US)
(74) Representative: Roques, Sarah Elizabeth

(56) References cited:
- EP-A- 0 967 217
- WO-A-00/43552
- WO-A-00/65352
- WO-A-98/20019
- WO-A-98/20020
- WO-A-99/27140
- WO-A2-01/36682
- US-A- 4 801 688
- US-A- 5 728 526
- US-A- 5 789 555
- US-B1- 6 197 503
- YANG M ET AL: "Cytotoxicity and DNA binding characteristics of dextran-conjugated doxorubicins", BBA - GENERAL SUBJECTS, ELSEVIER SCIENCE PUBLISHERS, NL, vol. 1380, no. 3, 8 May 1998 (1998-05-08), pages 329-335, XP004276114, ISSN: 0304-4165
- COYNE C P ET AL: "INHIBITION OF LIPOPOLYSACCHARIDE-INDUCED TNF-ALPHA PRODUCTION BY SEMISYNTHETIC POLYMYXIN-B CONJUGATED DEXTRAN", BIOTECHNOLOGY THERAPEUTICS, MARCEL DEKKER, NEW YORK, NY, US, vol. 5, no. 3/4, January 1994 (1994-01), pages 137-162, XP000605446, ISSN: 0898-2848
- GAUDIN V ET AL: "DETERMINATION OF SULFAMETHAZINE IN MILK BY BIOSENSOR IMMUNOASSAY", JOURNAL OF AOAC INTERNATIONAL, AOAC INTERNATIONAL, ARLINGTON, VA, US, vol. 82, no. 6, 1999, pages 1316-1320, XP009056399, ISSN: 1060-3271
- MEHVAR R: "Dextrans for targeted and sustained delivery of therapeutic and imaging agents", JOURNAL OF CONTROLLED RELEASE, ELSEVIER, AMSTERDAM, NL, vol. 69, no. 1, 3 October 2000 (2000-10-03), pages 1-25, XP004217531, ISSN: 0168-3659
- VO-DINH T ET AL: "DNA BIOCHIP USING A PHOTOTRANSISTOR INTEGRATED CIRCUIT", ANALYTICAL CHEMISTRY, AMERICAN CHEMICAL SOCIETY. COLUMBUS, US, vol. 71, no. 2, 15 January 1999 (1999-01-15), pages 358-363, XP000996839, ISSN: 0003-2700
- VO-DINH T: "DEVELOPMENT OF A DNA BIOCHIP: PRINCIPLE AND APPLICATIONS", SENSORS AND ACTUATORS B, ELSEVIER SEQUOIA S.A., LAUSANNE, CH, vol. B51, no. 1/2/3, 31 August 1998 (1998-08-31), pages 52-59, XP000669813, ISSN: 0925-4005
- WATTS H J ET AL: "REAL-TIME DETECTION AND QUANTIFICATION OF DNA HYDRIDAZATION BY AN OPTICAL BIOSENSOR", ANALYTICAL CHEMISTRY, AMERICAN CHEMICAL SOCIETY, US, vol. 67, no. 23, 1 December 1995 (1995-12-01), pages 4283-4289, XP000540115, ISSN: 0003-2700, DOI: DOI:10.1021/AC00119A013

## Description

### TECHNICAL FIELD

This invention relates to conjugate probes and optical detection of analytes. More specifically, this invention relates to conjugate probes which are used to form an array for a sensor or system employing optical detection of analytes.

### BACKGROUND OF THE INVENTION

Biomolecules and other analytes can be detected using arrays or microarrays of selective or specific probes which bind target analytes. Schemes have been developed in biosensor array technology to arrange probe spots on substrates or biochips. Arrays are used to detect and discover gene sequences, to select and test drug molecule candidates, to investigate toxicological or pharmacological action, and other uses. Targets may bind to probes of an array through a variety of interactions, including nucleic acid base pairing or hybridization, protein-protein interactions, protein-ligand interactions, enzyme-substrate interactions, receptor-ligand interactions, and other chemical reactions.

Bio-sensors allow simultaneous examination of a large number of interactions between biomolecules, such as proteins or nucleic acids, in a microarray format. They represent a powerful tool in utilizing the large amount of sequence information generated from the Human Genome Project, as well as that from genome sequencing of other organisms.

The signal from analyte species is generally small, and background arising from various sources makes the signal-to-noise ratio of the measurement relatively low. Low signal translates to low signal-to-noise ratio and poor detection of analytes. A solution is to enhance the signal from analytes, to increase the inherent signal-to-noise ratio of the detection. Increasing the signal-to-noise ratio lowers the detection limit for analytes, making it possible to observe analytes at lower concentration, opening new doors for applications to biomolecules.

WO98/20019 A relates to functionalized surfaces comprising immobilised beads.

Vo-Dinh et al. (1999) Anal. Chem. 71, 358-363 relates to the preparation of biosensors based on CCD chips.

Vo-Dinh (1998) Sensors and Actuators B51, no. 1/2/3 relates to the preparation of biosensors based on CMOS chips.

WO00/43552 A relates to multifunctional and multispectral biosensor devices.

US 5 789 555 A relates to an immobilised labelling method.

### SUMMARY OF THE INVENTION

This invention relates to an array for detecting analytes with an image sensor, the array comprising:
- a complementary metal-oxide-semiconductor (CMOS) optical digital image sensor comprising as its top layer a transparent passivation layer; and
- a plurality of conjugate probes linked covalently to said transparent passivation layer;
wherein at least one conjugate of the plurality of conjugates comprises a target-binding probe moiety covalently coupled to a polymer.

In another aspect, this invention relates to a method of detecting analytes, the method comprising contacting a fluid containing target analytes, with an array of the invention.

### BRIEF DESCRIPTION OF THE DRAWINGS

Fig. 1 illustrates an embodiment of a low-light image sensor enclosure attached to an embodiment of a reading station.
Fig. 2 illustrates a side view of an embodiment of a low-light image sensor enclosure.
Fig. 3 illustrates an embodiment of a biosensor system with removable optical image sensor.
Fig. 4 illustrates an embodiment of the detection of array probe spot signals using a CMOS image sensor.

### DETAILED DESCRIPTION OF THE INVENTION

An "array" or "microarray" is a linear or two-dimensional matrix or array of discrete regions, each having a defined area, formed on the surface of a solid support. The discrete regions may or may not overlap. The density of the discrete regions in a microarray is determined by the total numbers of target molecules, such as polynucleotides, to be detected on the surface of a single solid phase support. Although two or more regions may form an array, the typically density of the discrete regions is at least about 50/cm², often at least about 100/cm, more often at least about 500/cm², and sometimes at least about 1,000%m². As used herein, a DNA microarray is an array of oligonucleotide primers placed on a chip or other surfaces used to amplify or clone target polynucleotides. Since the position of each particular group of primers in the array is known, the identities of the target polynucleotides can be determined based on their binding to a particular position in the microarray.

The term "label" or "label species" refers to a composition capable of producing a detectable signal indicative of the presence of the target polynucleotide in an assay sample. Suitable labels include radioisotopes, nucleotide chromophores, enzymes, substrates, fluorescent molecules, chemiluminescent moieties, magnetic particles, nanoparticles such as quantum dots, and bioluminescent moieties. As such, a label is any composition detectable by spectroscopic, photochemical, biochemical, immunochemical, electrical, optical or chemical means.

As used herein, a "biological sample" refers to a sample of tissue or fluid isolated from an individual, including but not limited to, for example, blood, plasma, serum, spinal fluid, lymph fluid, the external sections of the skin, respiratory, intestinal, and genitourinary tracts, tears, saliva, milk, cells (including but not limited to blood cells), tumors, organs, and also samples of in vitro cell culture constituents.

The term "biological sources" as used herein refers to the sources from which the target polynucleotides are derived from. The source can be of any form of "sample" as described above, including but not limited to, cell, tissue or fluid. "Different biological sources" can refer to different cells/tissues/organs of the same individual, or cells/tissues/organs from different individuals of the same species, or cells/tissues/organs from different species.

A "polynucleotide" is a polymeric form of nucleotides of any length, either ribonucleotides or deoxyribonucleotides. This term refers only to the primary structure of the molecule. Thus, this term includes double- and single-stranded DNA and RNA, and forms such as A-, B-, H- and Z-form DNA and triplex forms. It also includes known types of modifications, for example, labels which are known in the art, methylation, "caps", substitution of one or more of the naturally occurring nucleotides with an analog, intemucleotide modifications such as, for example, those with uncharged linkages (e.g., phosphorothioates, phosphorodithioates, etc.), those containing pendant moieties, such as, for example proteins (including for e.g., nucleases, toxins, antibodies, signal peptides, poly-L-lysine, etc.),those with intercalators (e.g., acridine, psoralen, etc.), those containing chelators (e.g., metals, radioactive metals, etc.), those containing alkylators, those with modified linkages (e.g., alpha anomeric nucleic acids, etc.), as well as unmodified forms of the polynucleotide.

As used herein, the terms biological "binding partners" or "ligand/antiligand" or "ligand/antiligand complex" refers to molecules that specifically recognize (e.g. bind) other molecules to form a binding complex such as antibody-antigen, lectin-carbohydrate, nucleic acid-nucleic acid, biotin-avidin, etc. Biological binding partners need not be limited to pairs of single molecules. Thus, for example, a single ligand may be bound by the coordinated action of two or more "anti-ligands".

As used herein, the term "ligand" or "analyte" or "marker" refers to any molecule being detected. It is detected through its interaction with an antiligand, which specifically or non-specifically binds the ligand, or by the ligand's characteristic properties, such as dielectric properties. The ligand is generally defined as any molecule for which there exists another molecule (i.e. an antiligand) which specifically or non-specifically binds to said ligand, owing to recognition of some portion of said ligand. The antiligand, for example, can be an antibody and the ligand a molecule such as an antigen which binds specifically to the antibody. In the event that the antigen is bound to a surface and the antibody is the molecule being detected, for the purposes of this document the antibody becomes the ligand and the antigen is the antiligand. The ligand may also consist of cells, cell membranes, organelles and synthetic analogues thereof.

Ligands to be used to practice this invention include, but are not limited to, antibodies (forming an antibody/epitope complex), antigens, nucleic acids (e.g. natural or synthetic DNA, RNA, GDNA, HDNA, cDNA, mRNA, tRNA, etc.), lectins, sugars (e.g. forming a lectin/sugar complex), glycoproteins, receptors and their cognate ligand (e.g. growth factors and their associated receptors, cytokines and their associated receptors, signaling receptors, etc.), small molecules such as drug candidates (either from natural products or synthetic analogues developed and stored in combinatorial libraries), metabolites, drugs of abuse and their metabolic by-products, co-factors such as vitamins and other naturally occurring and synthetic compounds, oxygen and other gases found in physiologic fluids, cells, cellular constituents cell membranes and associated structures, other natural products found in plant and animal sources, other partially or completely synthetic products, and the like.

As used herein, the term "binding event" refers to an interaction or association between at least two molecular structures, such as a ligand and an antiligand. The interaction may occur when the two molecular structures as are in direct or indirect physical contact or when the two structures are physically separated but electromagnetically coupled therebetween. Examples of binding events of interest include, but are not limited to, ligand/receptor, antigen/antibody, enzyme/substrate, DNA/DNA, DNA/RNA, RNA/RNA, hybrids, nucleic acid mismatches, complementary nucleic acids and nucleic acid/proteins. Alternatively, the term "binding event" may refer to a single molecule or molecular structure described herein, such as a ligand, or an antiligand/ligand complex, which is bound to the signal path. In this case the signal path is the second molecular structure.

As used herein, the term "ligand/antiligand complex" refers to the ligand bound to the antiligand. The binding may be specific or non-specific, and the bonds are typically covalent bonds, hydrogen bonds, immunological binding, Van der Waals forces, ionic forces, or other types of binding.

As used herein, the term "coupling," with respect to molecules and molecular moieties, refers to the attachment or association of molecules, whether specific or non-specific, as the result of chemical reaction, or as the result of direct or indirect physical interactions, van der Waals interactions, London forces, or weak interactions, or as the result of magnetic, electrostatic, or electromagnetic interaction.

### Conjugate probes

In general, the probes of an array capture and bind the target analyte to be detected. Probe capture or target-binding moieties include nucleic acids, polynucleotides, proteins, peptide nucleic acids, small molecules, and a wide variety of biomolecules. Target-binding probe moieties include an epitope-binding domain of an antibody.

In one aspect, this invention embodies increased numbers of analytes within each point or spot of the array. The increased number of analytes per spot is achieved by compositions of conjugate probes which can capture enhanced numbers of analyte moieties. Conjugate probes including, for example, polysaccharide conjugates, are used to bring enhanced numbers of analyte moieties to the spot points of the microarray.

In one embodiment, conjugates are formed from a polymer linked to chemical or biomolecules, where the chemical or biomolecules include a probe or probes, thereby forming conjugate probes. The chemical or biomolecules containing the probe or probes are coupled to the polymer by covalent bonds, or by non-covalent chemical interactions such as ionic interactions or weak binding forces. The polymer may be a linear or branched polymer, such as a linear or branched polysaccharide or oligonucleotide, for example.

The polymer can be a solid, gel or amorphous composition, in the form of layers, beads, discs or mixtures thereof, and can be homogeneous or heterogeneous, linear or branched, side-chain branched, branched comb, or star or dendrimeric. Polymer branches may be long-chain branches or short-chain branches. The polymers are made by synthetic methods, or may be obtained as natural products isolated from naturally-occurring sources. Examples of the polymer include carbohydrates, saccharides, homopolysaccharides, heteropolysaccharides, agarose, amylose, amylopectin, glycogen, dextran, cellulose, chitin, chitosan, peptidoglycan, and glycosaminoglycan. In some embodiments, the polymer is a highly branched dextran. In further embodiments, the polymer is a hydrated dextran or agarose, such as a hydrogel, or is a polyacrylamide, gel. Further examples of the polymer used to make the conjugates include oligonucleotides, peptides, peptide nucleic acids, proteoglycans, glycoproteins, and glycolipids. In further embodiments, the polymer can be an antibody or antibody fragment.

Further examples of polymers useful for making conjugates include diol-containing polymers, such as polymers having gem-diol and vicinal-diol groups. Another example is a polymer having a hydroxyl group vicinal to an ester group, such as a phosphodiester linkage in an RNA.

Further examples of polymers are synthetic or naturally-occurring polynucleotides such as poly(T), poly(A), or poly(U).

Another example is a polymer having a plurality of hydroxyl groups. Mixtures of any of these polymeric species may be used in an embodiment of this invention.

The conjugate probes are prepared by coupling the chemical or biomolecule to the polymer. Examples of general methods to prepare conjugates are reviewed in Greg T. Hermanson, Bioconjugate Techniques (Academic Press 1996). In some embodiments, the conjugate probes are prepared by a conjugation reaction of a functional or reactive group on the chemical or biomolecule with the polymer, which couples the chemical or biomolecule to the polymer. Functional or reactive groups on the chemical or biomolecule include, for example, aldehydes, hydroxyls, amines or amino groups, carboxylates, sulfhydryl groups, and mixtures thereof.

The conjugate probes are prepared by coupling or reacting a chemical or biomolecule with the polymer, where the polymer may be derivatized to contain a plurality of sites for attachment to the functional or reactive groups of the chemical or biomolecules, either directly, or indirectly via linker groups. The derivatized polymer has reactive groups which can be used to attach chemical or biomolecules. The reactive groups of the derivatized polymer may be aldehydes, hydroxyls, amines or amino groups, carboxylates, sulfhydryls, isothiocyanates, N-hydroxysuccinimide esters, ketones, glyoxals, epoxides, oxiranes, imidoesters, carbodiimidazoles, alkylphosphates, anhydrides, maleimides, aziridines, acryloyls, fluorophenyls, diazoacetyls, N-acylimidazoles, succinimidyl carbonates, carboxymethyl groups, isocyanates, hydrazide groups, acrylazides, and mixtures thereof.

The polymer may have a reactive amine group such as the amino group in chitosan. In further embodiments, the polymer has reactive functional groups such as sulfates, carboxylates, or phosphate groups. Examples of sulfate-containing polymers include chondroitin sulfate, dermatan sulfate, heparin sulfate and keratin sulfate. Examples of carboxylate-containing polymers are polysaccharides containing groups which are derivative of sialic acid, aldonic acid, uronic acid, oxoaldonic acid, and ascorbic acid.

Examples of phosphate-containing polymers include nucleic acids such as DNA or RNA. These polymers may be conjugated to a chemical or biomolecule to make a conjugate probe using bifunctional linkers such as homobifunctional, heterobifunctional, or multifunctional linkers. For example, the conjugate probe may be a polynucleotide polymer conjugated to another polynucleotide. In one example, RNA is oxidized to provide aldehyde groups for attachment to chemical or biomolecules to make a conjugate.

A variety of chemical or biomolecules may be coupled to the polymer to provide conjugate probes capable of binding a variety of targets. In other words, a single polymer chain may be coupled to a variety of chemical or biomolecules to provide a conjugate probe. Mixtures of conjugate probes may be used in an embodiment of this invention.

In one embodiment, to prepare a conjugate probe amino groups on each of the polymer and the chemical or biomolecule are linked using dithiobis(succinimidylpropionate), disuccinimidyl tartarate, or disuccinimidyl glutarate. In further embodiments, a sulfhydryl group of the chemical or biomolecule is linked with an amine group of the polymer using N-succinimidyl 3-(2-pyridyldithio)propionate or *m*-maleimidobenzoyl-N-hydroxysuccinimide ester. In another embodiment, a sulfhydryl group of the chemical or biomolecule is linked with an aldehyde group of the polymer using 4-(N-maleimidomethyl)cyclohexane-1-carboxyl-hydrazide or 3-(2-pyridyldithio)propionyl hydrazide. In a further embodiment, a sulfhydryl group of the chemical or biomolecule is linked with a carboxylate group of the polymer using 4-(p-azidosalicylamido)butylamine.

Amino groups on each of the polymer and the chemical or biomolecule may be linked in further embodiments using heterobifunctional crosslinkers N-5-azido-2-nitrobenzoyloxysuccinimide or N-hydroxysulfosuccinimidyl-4-azidobenzoate.

In one embodiment, the conjugation is performed by reacting the hydroxyl groups of the polymer with a carbonylating agent such as N,N'-carbonyldiimidazole to form an intermediate imidazolyl carbamate, which in turn, can react with N-nucleophiles such as amines, amino-containing moieties such as peptides and proteins, to give an N-alkyl carbamate linkage.

In another embodiment, the conjugation is performed by reacting the hydroxyl groups of the polymer with N,N'-disuccinimidylcarbonate, followed by reaction with an amino-containing moiety, such as, for example, an amino group on an oligonucleotide. The amino group may be a terminal amino group or proximal to a terminus of the oligonucleotide.

The conjugation may be performed by reacting the polymer with 3-maleimidopropionic acid, followed by reacting the product, a derivatized polymer, with an amino group on an oligonucleotide.

In another embodiment, the conjugation is performed by reacting polymer hydroxyl groups with alkyl halide terminal groups of the chemical or biomolecule to give ether linkages in the probe conjugate.

Polymers containing hydroxyl groups on adjacent carbon atoms, for example saccharides or glycoproteins, may be reacted with sodium periodate to produce aldehyde functional groups on the polymer that can be used to conjugate chemical or biomolecules to prepare conjugate probes. Subsequent reaction of the aldehyde functional groups on the polymer with an amine-containing chemical or biomolecule produces a Schiff's base linkage between the polymer and the molecule. The Schiff's base linkage can be reacted with reducing agents such as sodium borohydride or sodium cyanoborohydride to produce a secondary or tertiary amine linkage between the polymer and the chemical or biomolecule.

In further embodiments, conjugate probes are prepared using photoreactive crosslinkers. For example, amino groups on each of the polymer and the chemical or biomolecule may be coupled to a photoreactive crosslinker, thereby forming a conjugate in which the polymer is coupled to the chemical or biomolecule through a linking group. In one example, an amino group of the polymer can be coupled to N-hydroxysuccinimidyl-4-azidosalicylic acid, and a amino group of the chemical or biomolecule may then be coupled by photolysis to form the conjugate in which the polymer is coupled to the chemical or biomolecule through a linking group.

In another example, the sulfhydryl group of the chemical or biomolecule may be coupled to 1-(p-azidosalicylamido)-4-(iodoacetamido)butane, and an amino group of the polymer may then be coupled by photolysis to form the conjugate in which the polymer is coupled to the chemical or biomolecule through a linking group.

In another example, the aldehyde group of the polymer may be coupled to p-azidobenzoyl hydrazide, and an amino group of the chemical or biomolecule may then be coupled by photolysis to form the conjugate in which the polymer is coupled to the chemical or biomolecule through a linking group.

In some embodiments, avidin-biotin interaction is used for the conjugation reaction. Reactive groups such as amino, carboxylate, sulfhydryl, and carbohydrates can be biotinylated. The biotin groups can be used to bind avidin or streptavidin, which may carry a label.

The coupling of conjugate probes to the surface of the sensor to make an array can be done in a number of ways. For example, the surface may be derivatized with an epoxide, which can react with reactive -OH or -NH₂- groups in the conjugate probes. In another example, the sensor surface is treated with poly(lysine), and conjugate probes or biomolecules are spotted onto the surface. UV irradiation may optionally be used to crosslink the conjugate probes or biomolecules to a substrate, such as a glass slide or passivation layer adjacent to an electronic device. Conjugate probes or oligonucleotides may be coupled to a sensor surface which has been derivatized with aldehyde, amine, or isothiocyanide groups. The mechanics for the formation of the array can be spotting, inkjet printing, or direct on-chip synthesis.

Additionally, the probes may be applied to a solid support using a robotic system, such as one manufactured by Genetic MicroSystems (Woburn, MA), GeneMachines (San Carlos, CA) or Cartesian Technologies (Irvine, CA), or Packard Bioscience (Billerica, MA).

The typical chemistry involved in attaching a ligand to the detection array layer will in general depend on the nature of the ligand and any antiligand to which it binds, and their functions in the assay. A list of possible types of interactions that may occur on the surface include but are not limited to: protein/protein interactions, DNA/protein interactions, RNA/protein interactions, nucleic acid hybridization, including base pair mismatch analysis, RNA/RNA interactions, TRNA interactions, enzyme/substrate systems, antigen/antibody interactions, small molecule/protein interactions, drug/receptor interactions, membrane/receptor interactions, conformational changes in solid phase ligands, protein/saccharide interactions, and lipid/protein interactions.

The actual surface chemistry may be described in one embodiment as primary binding and secondary binding. Additional regions of molecular binding may also occur. Primary binding refers to the attachment of an antiligand to the detection surface, which can be done through the assistance of a linker molecule.

In one embodiment, the invention provides a prepared solid support comprising immobilized or non-immobilized, separate groups of oligonucleotide probes. The probes can be selected or designed using for example a standard polymerase chain reaction (PCR) probe selection program such as Probe3 from Massachusetts Institute of Technology.

The solid phase support can provide an areas of about 5 to about 100 square micrometers, on which up to about 100,000 groups of probes can be immobilized in discrete areas according to a predetermined pattern. The prepared solid support can have an associated written or electronic record of the sequence of the probe or probe pairs at any given location on the support, and thus the location on the support of an amplified target can be identified as well.

The number of probes within each group corresponding to a particular region of a reference sequence can be determined and limited by the needs of the subsequent planned amplification reaction on the microarray. Thus, for example, the number of probes deemed necessary for conducting a PCR amplification at a specific site on the microarray, given especially the reaction volume and expected number of target template polynucleotide molecules, and the proposed number of cycles of PCR, will help determine exactly how much oligonucleotide probe copies to apply as a group at each location on the support to ensure successful reactions. Sometimes, the amounts of probes (i.e. probe molecule numbers or probe concentration) will be about the same at each location on a given solid support (e.g. in a DNA microarray format having from 1000, to 10,000, up to about 100,000 groups of probes to amplify or detect up to about 100,000 regions of the target polynucleotide).

It is understood that any nucleic acid containing probe may contain minor deletions, additions and/or substitutions of nucleic acid bases.

Oligonucleotide probes include the naturally-occurring heterocyclic bases normally found in nucleic acids (uracil, cytosine, thymine, adenine and guanine), as well as modified bases and base analogues. Any modified base or base analogue compatible with hybridization of the probe to a target sequence is useful in the practice of the invention.

The sugar or glycoside portion of the polynucleotide probe can comprise deoxyribose, ribose, and/or modified forms of these sugars, such as, for example, 2'-O-alkyl ribose. In a preferred embodiment, the sugar moiety is 2'-deoxyribose; however, any sugar moiety that is compatible with the ability of the probe to hybridize to a target sequence can be used.

In one embodiment, the nucleoside units of the probe are linked by a phosphodiester backbone, as is well known in the art. In additional embodiments, internucleotide linkages can include any linkage that is compatible with specific hybridization of the probe including, but not limited to phosphorothioate, methylphosphonate, sulfamate (*e.g.,* U.S. Patent No. 5,470,967) and polyamide (*i*.*e.,* peptide nucleic acids). Peptide nucleic acids are described in Nielsen et al. (1991) Science 254: 1497-1500, U.S. Patent No. 5,714,331, and Nielsen (1999) Curr. Opin. Biotechnol. 10:71-75.

In certain embodiments, the probe can be a chimeric molecule; *i.e.,* can comprise more than one type of base or sugar subunit, and/or the linkages can be of more than one type within the same probe.

The probe can comprise a moiety to facilitate hybridization to its target sequence, as are known in the art, for example, intercalators and/or minor groove binders.

Variations of the bases, sugars, and internucleoside backbone, as well as the presence of any pendant group on the probe, will be compatible with the ability of the probe to bind, in a sequence-specific fashion, with its target sequence. A large number of structural modifications, both known and to be developed, are possible within these bounds. Moreover, synthetic methods for preparing the various heterocyclic bases, sugars, nucleosides and nucleotides which form the probe, and preparation of oligonucleotides of specific predetermined sequence, are well-developed and known in the art. One method for oligonucleotide synthesis incorporates the teaching of U.S. Patent No. 5,419,966.

The oligonucleotide probes can be designed with any special additional moieties or sequences that will aid and facilitate a particular PCR or subsequent manipulations, e.g. isolation of the amplified target polynucleotides. For example, a probe can comprise sequences in addition to those that are complementary to the target sequence. Such sequences are normally upstream (*i*.*e*., to the 5'-side) of the target-complementary sequences in the probe. For example, sequences comprising one or more restriction enzyme recognition sites (so-called "linkers" or "adapters"), when present in a probe upstream of target-complementary sequences, facilitate cloning and subsequent manipulation of an amplification product. Other useful sequences for inclusion in a probe include those complementary to a sequencing probe and those specifying a promoter for a bacteriophage RNA polymerase, such as, for example, T3 RNA polymerase, T7 RNA polymerase and/or SP6 RNA polymerase.

In one aspect of the invention, the microarray probes are defined by a tiling method to cover an entire region of interest in the target polynucleotide. For example, a first group of probes are designed so that the sequence of each probe therein corresponds to the most 5'-portion of the region of interest; a second group of probes have sequence that is "shifted" from the first group by one nucleotide towards the 3'-end of the region; and a third group of probes have sequence that is "shifted" from the second group by one nucleotide toward the 3'-end of the region, and etc. In theory, then, the number of groups of probes equals the number of nucleotides in the region of interest. Of course, within each group of probes that correspond to a particular portion of the region, there are at least four sets of probes with four different 3'-ends as described above. When multiple target polynucleotides are to be detected according to the present invention, each probe group corresponding to a particular target polynucleotide is resided in a discrete area of the microarray.

### Digital image biosensor system

In one aspect of this invention, detection of analytes with a biosensor system is enhanced by using digital image or "machine vision" sensing technology which can be used to read out the signal from the analyte bound to the array with less background, and correspondingly higher signal-to-noise ratio. In one embodiment, the biosensor system employs digital image sensing technology including a digital image sensor on a daughterboard, an array of conjugate probes, a low-light enclosure for the sensor which may provide thermal cooling for the sensor, and methods of integrating analyte signals.

In one embodiment, optical signal from an array comprising capture species such as conjugate probes is detected using a digital image sensor. The digital image sensor includes a matrix of photosensor elements. The conjugate probes may be spotted in an array on the digital image sensor and may be linked either covalently or non-covalently to a surface of the digital image sensor. In alternative embodiments, the array is spotted on a glass slide which can be placed adjacent to the digital image sensor. In such embodiments, a fiber optical coupler is optionally located between the glass slide and the sensor.

The localized region of each discrete probe spot in the array may be larger than that of an individual photosensor element in the digital image sensor. Typically, there does not exist a one-to-one correspondence between probe spots and individual photosensors. Alternatively, the probe spots may be about the same size as an individual photosensor element.

The digital image sensor is a complementary metal-oxide-semiconductor (CMOS) image sensor. Each sensor of the CMOS image sensor includes a photodiode cell which is linked to its own analogue to digital converter, amplifier, and register. The top layer of the CMOS image sensor is a passivation layer, which may be silicon dioxide substantially transparent to light, and serves as a fluid barrier to protect the semiconductor circuitry from the analyte solution to be delivered to the array. The passivation layer can also be a conducting transparent material.

In one aspect, this invention relates to enhancement of signal from detected species by detection of the signal with a digital image sensor, in which the array is formed directly on the digital image sensor. In one embodiment, optical detection of analyte chemiluminescence emission is performed with an array formed on a thin passivation layer on top of a digital image sensor. In this embodiment, signal is advantageously enhanced by the proximity of the array to the photosensitive elements of the digital image sensor.

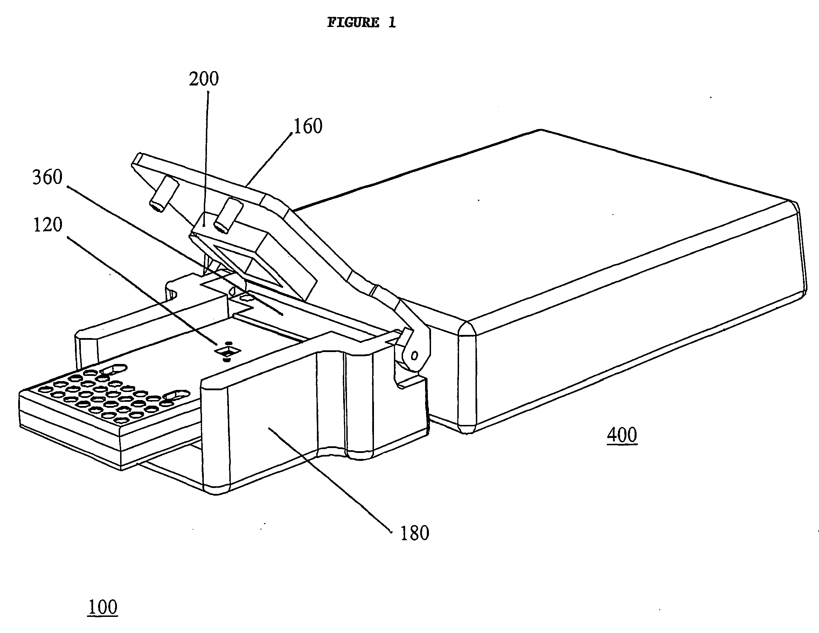

In another aspect of this invention, the sensor device or system provides increased signal-to-noise ratio of the measurement of analyte array light signals by reducing the background radiation impinging on the image sensor detector. As illustrated in the embodiment of Fig. 1, a low-light enclosure **100** is provided to contain the optical image sensor and the array. The enclosure **100** has a top shell **160** and a bottom shell **180.** The bottom shell **180** supports a printed circuit board for the optical image sensor, optional cooling elements for the sensor, and mechanically receives the top shell **160.** In the embodiment of Fig. 1, the printed circuit board for the optical image sensor is contained within a second enclosure **150.** The circuit board and the second enclosure are attached to the edge connector **360.** The top shell **160,** when received by the bottom shell **180,** provides a low-light region **300** defined by a barrier **200** which sealingly surrounds the array **120.** Fluid contacts the array in the low-light region defined by the barrier **200.**

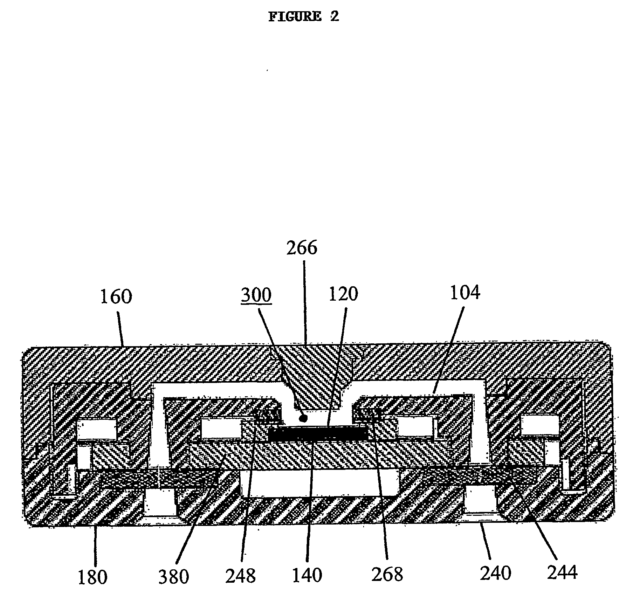

In another embodiment illustrated in Figure 2, a fluid entry opening **240** is provided in the bottom shell **180.** In operation, fluid is charged to the array **120** by injecting a liquid containing target molecules through the fluid entry opening **240.** The fluid pools in the low-light region **300.** Optionally, a capillary structure or fluid channel **104** is formed within the enclosure **100** to deliver the analyte from the fluid entry opening **240** to the array **120.** The fluid entry opening **240** optionally includes a septum **244** through which fluid is introduced, the septum being a barrier for both fluids and light.

In some alternative embodiments, for example when fluorescence detection is used, it is not required to have a fluid present in the low-light region **300.** In these embodiments, a fluid may be injected at the fluid entry opening **240** to provide cooling of the image sensor.

In the embodiment of Fig. 2, an optional lens is provided in the top shell **160** adjacent to the low-light region **300.** A sealant **268** such as foamed elastomer or an o-ring may also be provided to assist sealing of the low-light region **300.**

The enclosure **100** is connected to the reading station **400** so that the array is substantially gravitationally level. Optionally, the enclosure may be attached to the reading station and operated in any gravitational orientation. In these optional embodiments, the fluid entry opening and enclosure may encapsulate the analyte fluid by surface tension and capillary effects in any orientation, to the extent that the analyte array signals may be read out.

As illustrated in the embodiments of Figs. 1 and 2, the printed circuit board **380** supporting the optical image sensor **140** is electrically connected to the reading station **400** through an electrical edge connector **360.** The optical image sensor **140** may be secured and partly encapsulated with epoxy **248** for stability and protection. Optionally, the bottom shell **180** provides opening(s) for accessing the electronic circuits of the optical image sensor **140.**

In one embodiment, a biosensor system is provided which integrates analyte signal to increase the signal-to-noise ratio of the detection of analytes. Integration may be performed by increasing the frame collection time of the detector for the light arising from the array. To integrate analyte signal, a method of data transfer is provided using a CMOS image sensor. A typical CMOS image sensor is a fast frame rate device which may be used in video camera applications. In one embodiment, the CMOS image sensor is operated in a far slower regime in order to integrate the array signal impinging on the sensor. In this embodiment, the integrated signal is stored in memory, the integration is repeated, and the rate of change of analyte signal over time is observed. The frame collection time of the CMOS image sensor may be controlled, for example, to integrate analyte detection by clearing all on-chip registers at time zero, and then collecting the analyte radiation signal for a fixed period of time. In some embodiments, individual photosensor elements of the image sensor perform integration simultaneously for different periods. Integration of the analyte signal increases its signal-to-noise ratio and enhances detection of analytes, allowing a lower concentration of analyte to be detected.

In one embodiment, analyte signal is enhanced by reducing the "dark current" noise inherent in the CMOS image sensor by cooling the sensor within the low-light enclosure. The sensor may be cooled by a thermoelectric element, by nozzle expansion or refrigeration cooling methods, or by immersion in cooled fluids. A reduction of noise by about one-half is observed by cooling the sensor by 7° C, and cooling the sensor to 4° C reduces noise by about ten-fold relative to room temperature. In some embodiments, a fluid, which may or may not contain sample molecules, is injected into the low-light enclosure to provide cooling for the sensor.

### Analyte array signal

Optical detection of the analyte bound to a conjugate probe includes detection by fluorescence, chemiluminescence, bioluminescence, colorimetric, absorbance, and quantum dot methods. Label species or signal molecules are attached to the polymer, or to the probe of the conjugate probes, or to the analytes in the target mixture. Examples of label species or signal molecules include radioisotopes, fluorescers, chemiluminescers, chemiluminophores, bioluminescers, enzymes, antibodies, and particles such as magnetic particles and quantum dots. Fluorescent dye molecules attached to a short amine-derivatized oligonucleotide may be used as a label species, where the amine group is coupled to the polymer of the conjugate. Signal molecules used for analyte detection include radiolabels, fluorescent dyes such as Cy3, Cy5, Alexa Fluor 488, fluorescein, rodamine, Texas red, rose bengal, dansyl chloride, ethidium bromide, aminonapthalenes, pyrenes, and porphyrins, chemiluminescent systems such as luminol, dioxetanes, acridinium phenyl esters, and ruthenium salts, chromophores and colorimetric probes such as colloidal gold, azo dyes, quinolines dyes, and cyanine dyes.

A variety of schemes for detection of analytes are described in M. Schena and R. W. Davis, DNA Microarrays: A Practical Approach (M. Schena ed., Oxford University Press 1999).

Examples of label species used include agonists and antagonists, toxins, epitopes, hormones, antibodies, peptides, enzymes, oligonucleotides, peptide-nucleic acids, lectins, carbohydrates, proteins and drugs. For example, enzymes used in ELISA assays may be used for fluorescence detection. Another example is fluorescent-labeled avidin or streptavidin.

In some embodiments, more than one type of label species is used to provide more than one method of detection for a particular analyte. The polymer of the conjugate probe may be coupled to a plurality of fluorescent and chemiluminescent label species, for example. As described above, in some embodiments, the conjugate probe is capable of binding more than one target. Thus, in some embodiments, a polymer of the conjugate probe may be coupled to a plurality of target binding molecules and a plurality of different label species.

For fluorescence detection, array spot excitation light may be provided by an LED panel adjacent to the array, or alternatively adjacent to the CMOS sensor enclosure. In the fluorescence method, a narrow-band filter may be used adjacent to the array, between the array spots and the photodiodes to remove the excitation signal from the read out signals of the array, and to select the emitted light for detection.

In another method, analyte signal may be read out to provide assay information by optical detection of chemiluminescence. Chemiluminescence arises from light generated by a chemical reaction, which can be detected by a broadband detector without a filter, such as a CMOS image sensor. Light from the array spot is detected directly, and the background signal is mainly due to ambient light and "dark current." The analytes may be derivatized with chemiluminescent tags. Alkaline phosphatase or horse radish peroxidase, for example, can be used for chemiluminescence detection. The efficiency of detection may depend, in part, on the efficiency of attachment of the tags selectively or specifically to the targets. The label may be either biotin or digoxigenin that can be recognized by an enzyme detection system, followed by chemiluminescent reaction that converts the energy released from a chemical bond cleavage to photons of a discrete wavelength. Molecules which stabilize light generated from bond cleavage or chemical reaction, also called chemiluminescence enhancers, may by used for chemiluminescence detection.

The ratio of the number of signal molecules or dye molecules to the number of probe molecules which are coupled to the conjugate probe, or to the polymer, may be varied substantially. In some embodiments, the ratio of signal molecules to probe molecules is at least 3,4, or 5. Often, the ratio of signal molecules to probe molecules is at least 6, 7, 8, or 9. Sometimes the ratio of signal molecules to probe molecules is at least 10, 20, 30, 40, 50, 60, 70, 80, 90, or 100. Various combinations of signal molecules and probes may be used to form the conjugate probes.

In some embodiments of this invention, the array signal may be detected by a digital image sensor. In other embodiments, detection may be achieved with a charge coupled device (CCD), photomultiplier (PMT) or avalanche photodiode. Measurement of the analyte can also be done, for example, in various array schemes by electrical conductance detection.

Another aspect of the present invention relates to the application of conjugate probes in industrial, environmental, biomedical and biotechnology fields. Conjugate probes of this invention can be used in analytical or diagnostic applications, and to detect analytes in solution, gas or solid phase. The conjugate probes may be incorporated and used in a biosensor, to detect organic, inorganic or environmental particles in an analyte, or in an aqueous solution, non-aqueous phase, or gaseous phase.

Detecting an amplified or labeled target polynucleotide can be done by methods used for labeled sequences including, for example, detecting labels that have been incorporated into the amplified or newly synthesized DNA strands. Thus, for example fluorescent labels or radiolabels can be detected directly. Other labeling techniques may require that a label such as biotin or digoxigenin that is incorporated into the DNA during strand synthesis be detected by an antibody or other binding molecule (e.g. streptavidin) that is either labeled or which can bind a labeled molecule itself, for example, a labeled molecule can be e.g. an anti-streptavidin antibody or anti-digoxigenin antibody conjugated to either a fluorescent molecule (e.g. fluorescein isothiocyanate, Texas red and rhodamine), or conjugated to an enzymatically activatable molecule. Whatever the label on the newly synthesized molecules, and whether the label is directly in the DNA or conjugated to a molecule that binds the DNA (or binds a molecule that binds the DNA), the labels (e.g. fluorescent, enzymatic, chemiluminescent, or colorimetric) can be detected by a variety of techniques including a laser scanner, a CCD camera, or X-ray film, depending on the label, or other appropriate means for detecting a particular label.

A target polynucleotides may be detected by using labeled nucleotides (e.g. dNTP-fluorescent label for direct labeling; dNTP-biotin or dNTP-digoxigenin for indirect labeling) incorporated into amplified DNA during the PCR amplification. For indirectly labeled DNA, the detection is carried out by fluorescence or other enzyme conjugated streptavidin or anti-digoxigenin antibodies. The PCR method typically employs detection of the polynucleotides by detecting incorporated label in the newly synthesized complements to the polynucleotide targets. For this purpose, any label that can be incorporated into DNA as it is synthesized can be used, e.g. fluoro-dNTP, biotin-dNTP, or digoxigenin-dNTP, as described above. PCR amplification conducted using one or more universal primers in solution provides the option to detect the amplified targets at locations on the solid support by detecting the universal primers. Thus, where more than one universal primer is used, target strands from different sources can be differentially detected on the solid support.

Further examples of suitable fluorescent labels include fluorescein (FITC), 5,6-carboxymethyl fluorescein, Texas red, nitrobenz-2-oxa-1,3-diazol-4-yl (NBD), coumarin, dansyl chloride, rhodamine, 4'-6-diamidino-2-phenylinodole (DAPI), and the cyanine dyes Cy3, Cy3.5, Cy5, Cy5.5 and Cy7. Often, the fluorescent labels are fluorescein (5-carboxyfluorescein-N-hydroxysuccinimide ester) or rhodamine (5,6-tetramethyl rhodamine). Sometimes the fluorescent labels for combinatorial multicolor coding are FITC and the cyanine dyes Cy3, Cy3.5, Cy5, Cy5.5 and Cy7. The absorption and emission maxima, respectively, for these fluors are: FITC (490 nm; 520 nm), Cy3 (554 nm; 568 nm), Cy3.5 (581 nm; 588 nm), Cy5 (652 nm: 672 nm), Cy5.5 (682 nm; 703 nm) and Cy7 (755 nm; 778 nm), thus allowing their simultaneous detection.

Labeled nucleotides are sometimes used for detection labels since they can be directly incorporated during synthesis. Examples of detection labels that can be incorporated into amplified DNA or RNA include nucleotide analogs such as BrdUrd (Hoy and Schimke, Mutation Research 290:217-230 (1993)), BrUTP (Wansick et al., J. Cell Biology 122:283-293 (1993)) and nucleotides modified with biotin (Langer et al., Proc. Natl. Acad. Sci. USA 78:6633 (1981)) or with suitable haptens such as digoxygenin (Kerkhof, Anal. Biochem. 205:359-364 (1992)). Fluorescence-labeled nucleotides are Fluorescein-isothiocyanate-dUTP, Cyanine-3-dUTP and Cyanine-5-dUTP (Yu et al., Nucleic Acids Res., 22:3226-3232 (1994)). A useful nucleotide analog detection label for DNA is BrdUrd (BUDR triphosphate, Sigma), and a useful nucleotide analog detection label for RNA is Biotin-16-uridine-5'-triphosphate (Biotin-16-dUTP, Boehringher Mannheim). Fluorescein, Cy3, and Cy5 can be linked to dUTP for direct labeling. Cy3.5 and Cy7 are available as avidin or anti-digoxygenin conjugates for secondary detection of biotin- or digoxygenin-labeled probes.

In one embodiment, the invention is used to detect binding of a molecular structure to the signal path. In this embodiment, a signal is propagated along the signal path. As it propagates, it couples to the bound structure and is modulated. Analysis of the modulated response indicates binding.

In another embodiment, the invention may be used to identify secondary binding. For example, primary binding may be the attachment of an antibody to the conductive surface. Secondary binding might involve the measurement of binding between the immobilized antibody and its antigen in solution. After primary binding has been detected as described in the previous paragraph, the solution containing the antibody is added to the bio-assay device and the response measured again. The response is compared to the primary binding response. A change would indicate that a binding event has occurred.

In another embodiment, the invention may be used in an array format. The sensor device or biosensor system will have multiple addressable sites, each of which has bound to it a specific antiligand. After delivering solution to the device, binding responses at each site will be measured and characterized. A device of this type may be used to measure and/or identify the presence of specific nucleic acid sequences in a sample. At each of the addressable sites a unique nucleic sequence is attached as the antiligand. Upon exposure to the sample, complementary sequences will bind to appropriate sites. The response at each site will indicate whether a sequence has bound. Such measurement will also indicate whether the bound sequence is a perfect match with the antiligand sequence or if there are one or multiple mismatches. This embodiment may also be used to identify proteins and classes of proteins.

In another embodiment, this invention may be used to generate a standard curve or titration curve that would be used subsequently to determine the unknown concentration of a particular analyte or ligand. For example, an antibody could be attached to the sensor device or biosensor system. The device could be exposed to several different concentrations of the ligand and the response for each concentration measured. Such a curve is also known to those skilled in the art as a dose-response curve. An unknown sample can be exposed to the device and the response measured. Its response can be compared with the standard curve to determine the concentration of the ligand in the unknown sample.

Often it is desirable to determine certain qualities of a given molecule. Examples in include determining the class to which a protein belongs, or which type of polymorphism a given gene or other nucleic acid sequence is. This may be done in a number of ways. Proteins are often classified by number and types of structural homologies, or particular substructures which are found in the same or similar classes of proteins. For example, G-Proteins commonly found in cell membranes and which mediate signal transduction pathways between the extracellular environment and the intra-cellular environment, always have a structure which traverses the cell membrane seven times. Such a structure is virtually definitive of a G-Protein. Other classes of proteins have similar structural homologies, and as such, any method which can distinguish one class of proteins from another on the bases of these homologies is of enormous use in many of the biomedical research fields. Given that the dielectric properties of a given molecule is determined entirely by the geometry of the charge distribution of said molecule, and further given that most proteins have a unique structure or geometry, then each protein may be uniquely determined by measuring the dielectric properties of the protein. Thus a simple dielectric signature, such as the ones generated by the present invention, may serve to uniquely identify a given protein, and further, may allow classification of the protein into some previously known class of proteins. A further refinement may be added to the classification methodology by using a group of antiligands on the bio-assay device which are specific for particular sub-structures of a given protein. For example, a group of antibodies which are specific for particular sub-structures such as domains may be utilized for the determination of the existence or absence of said sub-structures. Thus, any given protein may be characterized by determining both the presence and absence of certain sub-structures as well as the dielectric properties of the protein itself. Further refinements to this classification strategy may include looking at temperature, pH, ionic strength, as well as other environmental effects on the above-mentioned properties.

Nucleic acids may also be characterized by following a similar paradigm. For example, a given gene may be known to have a certain base pair sequence. Often times in nature there will be small variations in this sequence. For example, in the gene which codes for a chloride ion transport channel in many cell membranes there are common single base-pair mutations, or changes. Such changes lead to a disease called cystic fibrosis in humans. Thus characterizing a given nucleic acid sequence with respect to small variations is of enormous importance. Such variations are often called polymorphisms, and such polymorphisms are currently detected by forming complementary strands for each of the known polymorphisms. Since any given gene may take the form of any one of hundreds or even thousands of polymorphisms, it is often an arduous task to generate complementary strands for each polymorphism. Using the invention described herein, non-complementary binding or hybridization may be detected and distinguished by measuring many of the same physical properties as were described in the previous paragraph: The dielectric properties of the hybridization event can be characterized and correlated to known data, thereby determining the type of hybridization which has occurred--either complete or incomplete. Thus with an antiligand comprised of a given nucleic acid sequence, hundreds of different polymorphisms (as ligands) may be detected by the characterization of the binding event. One of skill in the art will appreciate that further refinements are possible, such as modifying the stringency conditions to alter the hybridization process, or varying the temperature and determining the melting point, which serves as another indicator of the nature of the hybridization process.

In one embodiment, drug-receptor interactions may be characterized to determine if a given binding event results in the receptor being turned on or off, or some other form of allosteric effect. For example, a given receptor may be used as an antiligand, and a known agonist may be used as the first ligand. The interaction is characterized according to a dielectric property, or other property. Compounds being screened for drug candidates may be tested for their binding properties with the receptor. For example,. a candidate which binds and yields a value of the property similar to the known agonist has a higher probability of being an agonist. This method may be used to characterize any type of target-receptor binding event of interest, or other classes of binding events.

In one embodiment, the conjugate probe serves as the recognition system for an immunosorbent assay similar to an ELISA assay, or an immunoblot assay. Conjugates of this embodiment have an antibody coupled to the polymer, which is the primary anitbody of the ELISA assay or immunoblot assay. A secondary antibody coupled to an enzyme, which catalyzes a reaction that forms a colored product, may be used to recognize the primary antibody, or some moiety of the conjugate. A substrate of the enzyme may be used to produce color for detection. In further embodiments, a kit may be prepared for an assay system, the kit being assembled using, for example, a polysaccharide conjugate which is coupled to at least one recognition molecule and at least one signaling molecule or label species. In these embodiments, the recognition molecule may be an antibody, or other chemical or biomolecule.

### Sensor system

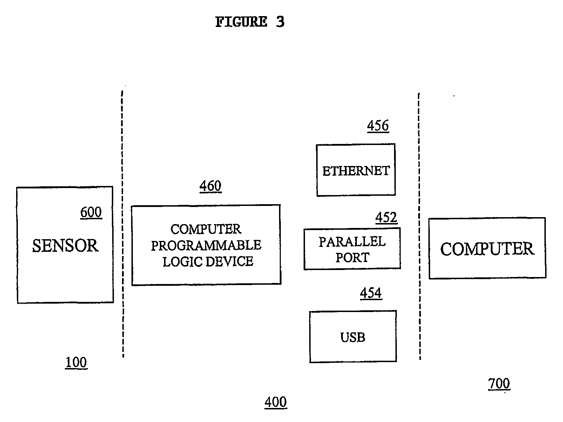

In one embodiment, as illustrated in Fig. 3, the biosensor system comprises a digital image sensor **600** in a low-light enclosure **100,** a high data throughput reading station **400,** and a general purpose computer **700.** The reading station **400** has at least one socket for inserting a low-light enclosure **100** containing a CMOS digital image sensor **600,** thereby electrically and mechanically connecting the sensor enclosure to the reading station. The reading station may be connected to the computer through universal serial bus (USB) **454,** for example, or by a different parallel port interface device **452.** Optionally, the reading station may be connected to the computer via Ethernet interface **456.** Other connections that can be used in the system include, but are not limited to, Firewire, SCSI, and PCMCIA. In alternative embodiments, the computer and the reading station can be replaced by a handheld computer, or a personal digital assistant such as a Palm Pilot.

Referring to the embodiment of Fig. 3, a programmable logic device **460** on a motherboard in the reading station **400** is interfaced to a general purpose computer **700,** such as a personal computer. The programmable logic device **460** is also interfaced to the digital image sensor **600,** and synchronizes the read out of digital image sensor analyte data by monitoring status lines from the digital image sensor which signal the start and end of images, including frame, line, and pixel data clock pulse lines. The programmable logic device **460** manages the flow of image data out of the image sensor **600** and into local FIFO memory, where the image is stored until the computer **700** requests a transfer of the data. A typical cycle for the programmable logic device **460** is to receive a command from the computer **700,** which causes the sensor to output an image, capture that image into local FIFO memory on the motherboard of the reading station **400,** and transfer the captured image data to the computer **700.** A graphical user interface (GUI) provides facility for the computer user to request an image capture and display cycle, a snap shot mode, or request a continuous sequence, a live video mode. Filters and image processing tools are provided to allow the user to operate the sensor under low-light conditions. These tools comprise image processing routines to boost small signals from the sensor, software routines to co-add images, routines to subtract background images or "dark" images, and routines to filter out noise. The GUI also gives the user control over the sensor-on-chip settings. This allows the user to interact with the sensor, adjusting on-chip parameters such as integration time, gain, and analog to digital converter range.

As exemplified by the embodiment of Fig. 3, the reading station **400** includes a connector **360** to attach the image sensor daughterboard and low-light enclosure **100** to the reading station **400.** A feature of the arrangement of this embodiment is that the optical digital image sensor analyte detector is readily mechanically separated from the reading station; in other words, it is removable. A removable digital image sensor advantageously provides portability of the digital image sensor, and high throughput operation of the biosensor system. In operation, the low-light enclosure and digital image sensor may be connected to the reading station from a queue, either manually or robotically, and after detecting analytes, the low-light enclosure and digital image sensor may be disconnected, either manually or automatically. This plug-and-play feature of the biosensor system allows operation of the biosensor system with a low-light enclosure and digital image sensor which is a disposable unit, for example. In alternative embodiments, the low-light enclosure and digital image sensor can be regenerated for use with a different array. In operation, the biosensor system reading station is provided with hot-swap capability, in which the low-light enclosure containing the optical image sensor can be connected to, and disconnected from the reading station without de-energizing the power supply of either the reading station or the image sensor.

The reading station includes a USB microcomputer interface. Optionally, a MICROSOFT EXTENDED CAPABILITIES PORT (ECP) interface may be included, with a user controlled switch to determine the active interface. The USB cable supplies electrical power which can be used by the reading station motherboard. For ECP, a 9 VDC supply is provided. A manual reset switch is provided to reset the biosensor motherboard, and the programmable logic device may also be manually reset.

In one embodiment, this invention is a method of enhancing analyte signal detection by time-integration. Data throughput and measurement of analyte parameters in the target are limited by the signal-to-noise inherent in the detection of light from the array by the digital image sensor. The signal-to-noise may be increased by integrating analyte signal for several milliseconds or longer, often from about 10 milliseconds to about two minutes, sometimes about 30 to about one thousand milliseconds, and sometimes about 50 to about 600 milliseconds. In another embodiment, the time dependence of analyte signal is recorded by storing a sequence of array signal frames, in which each frame is obtained by integrating analyte signal for a period of time.

The USB microcomputer interface provides the master clock for the image sensor and programmable logic device. The image sensor output includes pixel data along with image line and frame pulses, which are passed back through the connector to the motherboard and sent into a FIFO memory. The frame pulse is used to reset the FIFO pointer, and the line pulse is used as the write enable for the FIFO. This arrangement stores pixel data in the FIFO starting with the upper left pixel as location 0 (zero) of the FIFO.

Once the image is in the FIFO, it can be read out by one of two interfaces. Referring to the embodiment of Fig. 3, ECP is provided in which the array image data is read into a parallel port (PP) 452, one pixel per read. The read starts when the PP **452** sends a reverse request. This causes the programmable logic device **460** to enable its output drivers to the PP **452.** Then the programmable logic device **460** asserts the per.clock. The PP **452** responds with per.ack. The clock ack sequence continues until the computer **700** has read a frame of pixels. The programmable logic device **460** uses PP **452** data bit zero as SDA, and PP **452** data bit 1 as SCL of the I2C bus.

In another embodiment, image data in the FIFO is read out by USB interface. Biosensor operation is enhanced by increasing the bandwidth of serial data transmission as compared to conventional USB transfer. In conventional USB transfer, a packet of data from the FIFO would be read, followed by an interval of time in which the FIFO loads the next packet to be read out. For example, in a conventional USB microcomputer interface a packet of 63 pixels is read from the FIFO and sent via one of the data lines in the USB. In one embodiment, two end points are designated in the FIFO to establish two buffers. In operation, one buffer is read out and transmitted on one of the data lines in the USB while the other buffer is being filled, thereby increasing the transfer bandwidth using the universal serial bus by up to 100%. The end of the data transmission from the first buffer occurs immediately before, for example, one or a few clock pulses before, the start of data transmission on a data line of the universal serial bus from the second buffer. Then data transmission on a data line of the universal serial bus from the second buffer occurs, while at the same time loading data into the first buffer. These steps may be repeated until all the data in need of transfer is sent, thereby increasing the data transfer rate over conventional USB.

The following examples further describe embodiments of the present invention. The examples are given solely for the purpose of illustration and are not to be construed as limiting the present invention. While there have been described illustrative embodiments of this invention, those skilled in the art will recognize that they may be changed or modified without departing from the spirit and scope of this invention, and it is intended to cover all such changes, modifications, and equivalent arrangements that fall within the true scope of the invention as set forth in the appended claims.

### EXAMPLES

### Example 1

Linear polysaccharide dextran (Sigma) was dissolved in deionized water to a final concentration of 1% and then autoclaved. An aliquot of 0.40 ml dextran solution was oxidized with 44 microliter of 0.5 M sodium periodate overnight in the dark at room temperature on a rocking platform. The oxidized dextran was then cleaned by precipitation twice with 0.3 M NaOAC and 2xVol of EtOH. The pellet was air-dried and redissolved in 0.4 ml of 5 mM NaPO₄ buffer, pH 7.2.

One microliter of the oxidized dextran was added to 7 microliters of 10 mM NaCO₃ (pH 9.0) and 2 microliters oligonucleotides (2 µM solution in H₂O) in an Eppendorff tube. The oligonucleotides varied in length from 25 to 45 mer, with a primary amine introduced at either the 3' or 5' end during synthesis. The reaction was carried out overnight in a 37°C water bath. NaBH₄ was added to the tube and the mixture was incubated further for 30 minutes at room temperature, then precipitated with 0.3 M NaOAc and 2xVol of EtOH. The pellet was dissolved in TE buffer and an aliquot was resolved on a 1% agarose gel by electrophoresis, and subsequently stained with EtBr. In this detection system, free oligonucleotide migrated close to the salt-front, while oligonucleotide coupled to dextran migrated much slower.

### Example 2

High-molecular-weight branched polysaccharides, glycogen (Sigma) and amylopectin (Sigma), were used to couple amine-derivatized oligonucleotides. Diol groups of these polymers were converted to aldehyde groups by oxidation with NaIO₄. Sodium periodate was added to 0.4 ml of 1% polysaccharide solution (in H₂O) to a final concentration of 25 mM for glycogen, and 20 mM for amylopectin, respectively. Oxidation was continued in dark overnight at room temperature on a rocking platform. The oxidized polysaccharide was then precipitated twice with 0.3 M NaOAc and 2xVol of EtOH to remove the excess NaIO₄. After air-drying, the pellets were dissolved in 0.4 ml of 5 mM NaPO₄ buffer (pH 7.2). Coupling of amine-derivatized oligonucleotide and gel-analysis of the coupled products were carried out as described in Example 1 for dextran.

### Example 3

The conjugate probe of Example 2 is prepared having, on average, about 1000 oligonucleotide molecules coupled to each glycogen molecule at a coupling density of one oligonucleotide per 10 glucose monomers.

### Example 4

Signaling molecule 5'-ACTGCT-3' (BP001) derivatized at the 5' end with amine and at the 3' end with fluorescent dye Cy5, and recognition probe molecule oligonucleotide AKH108 (5'-CCGTGCAGATCTTAATGTGCCAGTAAAAG-3 ') derivatized at the 5' end with an amine group are coupled to the same polysaccharide. AKH108 hybridizes to a PCR product amplified with the primers of 5'-CCGTGCAGATCTTAATGTGC-3' and 5'-GCGCTGTACCAAAGGCATC -3' from the bacterium Haemophilus influenzae genome, which corresponds to a fragment within the gene encoding 3-phosphoglycerate kinase. The PCR product is spotted onto a glass slide coated with poly(Lysine) in a mircoarray format.

For co-cross linking, 0.2 nmoles of AKH108 and 2 nmoles of BP001 are added to a tube containing 20 nmoles of oxidized glycogen in 10 mM NaCO₃ with a final volume of 10 microliters. The reaction is carried out at 37°C overnight. Then NaBH₄ is added to the tube to a final concentration of 4 mM and incubated at room temperature for another 90 minutes. The final products are precipitated with EtOH. After centrifugation, the cross-linked products as well as free AKH108 come down in the pellet, while free BP001 remains in the supernatant and is discarded. The pellet is dissolved 10 µl 3XSSPE/0.1% SDS/1.0 mg/ml BSA and is applied to the microarray surface. After hybridization at room temperature for five hours, the slide is washed 3 times with 10 µl of fresh 0.1XSSPE/0.1% SDS, and scanned in a laser scanner, and the spotted pattern for the PCR product is observed.

### Example 5

Oligonucleotide 5'-NH₂-CCGATGCCTTAGTTTCAA-GTGGTGCGATTGACATCGTTGTCAT-3', which specifically hybridizes to a PCR product amplified from the RecA gene from *Enterococcous faecalis*, was used to cross link to glycogen and amylopectin. The polysaccharides were oxidized as described in Example 2. For cross linking, 20 nmoles of the oxidized sugar and 2 nmoles of the amine derivatized oligonucleotides were mixed in 10 mM NaCO₃ buffer (pH 9.0) in a final volume of 10 µl, and then incubated at 37°C overnight. At the end of the coupling reaction, NaBH4 was added to a final concentration of 4 mM and incubated for another 60 minutes at room temperature. The final products were precipitated with 0.3 M NaOAc and 2xVol of EtOH, then dissolved in 10 mM NaCO₃ (pH 9.0). Aliquots of the cross linked oligonucleotides were used to spot onto Epoxy treated glass slide. An equivalent amount of the oligonucleotides mixed with the unoxidized polysaccharide was also spotted onto the same slide as a control. For on-chip hybridization, the RecA PCR product was labeled with Alexa Fluor 546 (Molecular Probes Inc), dissolved in 3xSSPE/0.1% SDS/1.0 mg/ml BSA, and applied to the glass slide surface. After three hours hybridization at room temperature and washes with 0.1xSSPE/0.1% SDS, the slide was scanned using a laser scanner. The spot signal for the oligonucleotide coupled to glycogen was 5.86 relative to the control unoxidized glycogen spot, and the spot signal for the oligonucleotide coupled to amylopectin was 5.63 relative to the control unoxidized amylopectin.

### Example 6

For carbodiimidazole (CDI) activation, 4 µl of 0.5 M CDI (in DMSO) was added to 10 µl of 0.5 % polysaccharide in DMSO and 6 µl of DMSO. The reactions were incubated at room temperature for three hours with occasional vortex. Then 1 ml of n-butanol was added to each tube, thoroughly mixed by vortexing, and spun in a microfuge. After removing the butanol, the pellets were air-dried and dissolved in 10 µl of DMSO. For oligonucleotide coupling, 2.2 nmoles of amine derivatized oligonucleotides were mixed with 5 µl of 0.5 % CDI-activated polysaccharide in a final volume of 20 µl. The reactions were incubated at room temperature for 5 days with occasional vortexing. At the end of the reactions, 1 µl of 10% ethanolamine was added and incubated further for 30 minutes, then EtOH precipitated. The products were then dissolved in 10 µl of TE buffer and analyzed by gel electrophoresis.

### Example 7

A CMOS image sensor was used for direct on-chip detection of hybridization signals.

The bare die of a PB0330 monochrome image sensor (Photobit) was attached to a daughter board with an edge connector. The bond wires were encapsulate in Epoxy and cured. The die surface was rinsed three times with autoclaved dH₂O and air-dried. To derivatize the surface with epoxy, a solution of 2% (3-glycidoxypropyl)trimethoxy-silane in methanol was applied to the die surface and incubated at room temperature for 10 minutes, then washed twice with methanol and air-dried.

Four different capture probes (100 pmoles/ml in 50 mM NaCO₃ buffer, pH 10.5) were manually spotted, in duplicate, onto the Epoxy treated die surface. After the spots dried, the surface was quickly washed once with 1% ethanolamine in 50 mM NaCO₃ (pH 10.5), and incubated in the same blocking solution for 10 minutes at room temperature. The surface was then rinsed four times with autoclaved dH₂O.

For on-chip hybridization, the die surface was incubated with 3xSSPE/50% formamide/1 mg/ml BSA for 20 minutes at room temperature, then hybridized with a PCR product (biotin label at one end, as described below) in 20 ml of 3xSSPE/50% formamide/1 mg/ml BSA (after it had been heated in a boiling water bath for 2 minutes and quickly cooled at 4°C). The hybridization was carried out at 30°C overnight in a moisturized chamber. Afterwards, the die surface was washed with 0.1xSSPE at room temperature four times, 5 minutes each. To bind streptavidin-alkaline phosphatase conjugate to the biotin on the hybridized PCR product, the die surface was first incubated with 1mg/ml BSA in TBS at room temperature for 20 minutes, then incubated with Avidx-AP (Tropix) at 1:100 dilution in TBS/1 mg/ml BSA for two hours at room temperature. The die surface was then washed with TBS five times, 5 minutes each at room temperature.

For detecting the on-chip hybridization signal, the daughter board with TBS on the die surface was inserted into the connector on the Reading Station in a light-proof enclosure. A proprietary software was launched on a PC to retrieve a "dark image" (i.e. the background image, I_{dark}). With the daughter board still attached to the Reading Station, the TBS was then replaced with a chemiluminescent substrate solution that include CDP-*star* and the enhancer Emerald II (both from Tropix) prepared according to the vendor's specifications. An image frame (I) with about 0.1 sec integration time was retrieved from the sensor. I - I_{dark} was treated as a real signal snapshot. The software has a built-in subroutine that adds successive processed snapshots together and displays the result as one image, thus further extending the signal integration time.

The following sensor register settings were found to be optimal at this point:
Gains (Registers 53, and 43-46) at the maximum; Integration time (Registers 9 and 10) at 0.1 second per frame; Analogue negative offset (Registers 32 and 57) at the maximum; Gainstage (Register 62) at 74. The rest of the Registers were left at the default setting.

The capture probes and PCR primers used in the experiment described below were the following:
Neisseria meningitidis
   Capture probe(1)
      5'-Amine-GGCAGAAGACGCGCTCAAACGTTACGGTTTTTCAGAC-3' Primers
      5'-GACGACTACGCCTTGGAC-3'
      5'-Biotin-AGACGGCGTAGTCTTCCAAA-3'
      Listeria monocytogenes
   Capture probe(2)
      5'-Amine-TGACCGCGTTGTAACAAAACACCCATTCTATGACCGTGATTC-3' Primers
      5'-GTCATCTGGACAACTACTCCTT-3'
      5'-Biotin-TGTCCAGGAGCTGTATGAAC-3'
      Hemophilus influenzae
   Capture probe (3)
      5'-Amine-CTTCTCACTTAGGTCGTCCAACTGAAGGAGAATTCAAACCAG-3' Primers
      5'-CCGTGCAGATCTTAATGTGCC-3'
      5'-Biotin-GCGCTGTACCAAAGGCATC-3'
      Mycoplasma pneumoniae
   Capture probe(4)
      5'-Amine-AAAGAGGAAACGCCAGCGGTGATCTTCCGTGG-3' Primers
      5'-GTTAATGGTGTTGGCAAAACAAC-3'
      5'-Biotin-AACCGTCCCGAGGTGTC-3'

The capture probes were cross-linked to oxidized glycogen as describe above. The final pellets were dissolved in 50 mM NaCO₃ (pH 10.5) at a final concentration of 100 picomoles/ml, and spotted onto epoxy-treated die surface in duplicate in the following pattern, where 1 = Neisseria meningitidis, 2 = Listeria monocytogenes, 3 = Hemophilus influenzae, 4 = Mycoplasma pneumoniae:

| | | | |
|---|---|---|---|
| 1 | 2 | 3 | 4 |
| 1 | 2 | 3 | 4 |

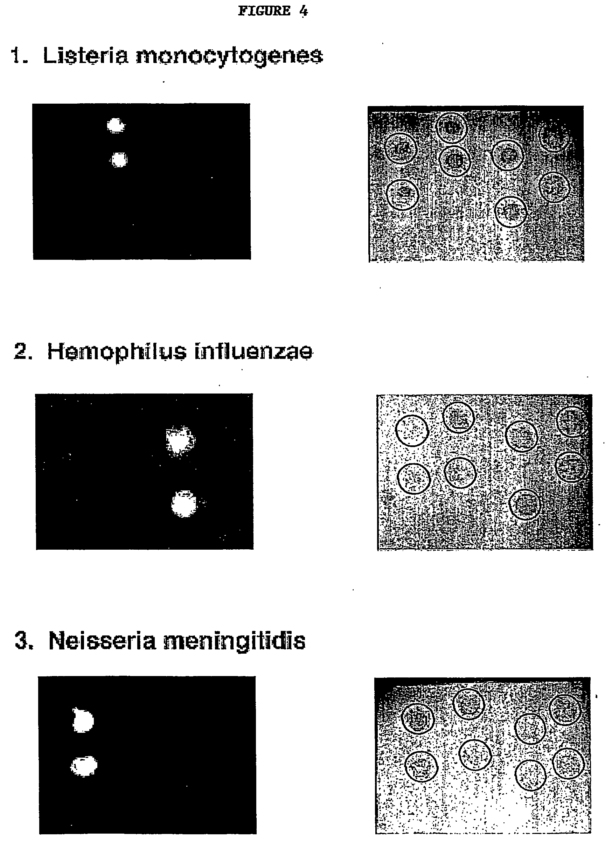

Fig. 4 shows the hybridization detection results using a CMOS image sensor. The integration time for each spot was about 0.5 seconds. For each analyte in Fig. 4, the image on the right shows the spotted array, and the image on the left shows the detected spots. The spots of the array illustrated on the right side of Fig. 4 are circled for convenience of view.
Also described herein are the following aspects:
1. A conjugate for use in detecting analytes with a sensor comprising a chemical or biomolecule coupled to a diol-containing polymer.
2. A conjugate for use in detecting analytes with a sensor comprising a chemical or biomolecule coupled to a linear polysaccharide.
3. A conjugate for use in detecting analytes with a sensor comprising a chemical or biomolecule coupled to a branched polysaccharide.
4. A conjugate for use in detecting analytes with a sensor comprising a chemical or biomolecule coupled to a polynucleotide having a hydroxyl group vicinal to a phosphodiester linkage.
5. A conjugate for use in detecting analytes with a sensor comprising a chemical or biomolecule coupled to a polymer selected from the group consisting of RNA, poly(T), poly(U), and poly(A).
6. A conjugate comprising a chemical or biomolecule coupled to a branched polysaccharide selected from the group consisting of amylose, amylopectin, glycogen, dextran, cellulose, chitin, chitosan, peptidoglycan, glycosaminoglycan, and mixtures thereof.
7. A conjugate as in any one of aspects 1-6 wherein the chemical or biomolecule is an oligonucleotide.
8. A conjugate as in any one of aspects 1-6 wherein the chemical or biomolecule is a protein nucleic acid.
9. A conjugate as in any one of aspects 1-6 wherein the chemical or biomolecule is a protein.
10. A conjugate as in any one of aspects 1-6 wherein the chemical or biomolecule is an antibody or antibody fragment.
11. A conjugate as in any one of aspects 1-6 comprising a plurality of chemical or biomolecules which are independently the same or different, and further comprising a plurality of label species which are independently the same or different.
12. A method of making a conjugate comprising,
   reacting a branched polysaccharide with an oxidizing agent, thereby forming a branched polysaccharide having aldehyde groups; and add
   ing a reactive amino group to each of a plurality of chemical or biomolecules; and
   coupling a plurality of the aldehyde groups to the plurality of chemical or biomolecules.
13. The method of aspect 12 wherein the oxidizing agent is sodium periodate.
14. The method of aspect 12 wherein the coupling of the plurality of aldehyde groups to at least one of the plurality of chemical or biomolecules is done with a photoreactive crosslinker.
15. A method of making a conjugate comprising,
   reacting a branched polysaccharide with a carbonylating agent, thereby forming a branched polysaccharide having carbamate groups; and addi
   ng a reactive amino group to each of a plurality of chemical or biomolecules; and
   coupling a plurality of the carbamate groups to the plurality of chemical or biomolecules.
16. The method of aspect 15 wherein the carbonylating agent is N,N'-carbonyldiimidazole or N,N'-disuccinimidylcarbonate.
17. A method of making a conjugate comprising reacting a branched polysaccharide with a heterobifunctional photoreactive crosslinker.
18. A method of making a conjugate comprising reacting a branched polysaccharide with 3-maleimidopropionic acid.
19. An array for detecting analytes with an image sensor comprising a conjugate as in any one of aspects 1-6.
20. An array as in aspect 19 wherein the conjugate is spotted on a surface of the image sensor.
21. An array as in aspect 19 wherein the conjugate is spotted on a substrate which is adjacent to the image sensor.
22. An array as in aspect 19 wherein the image sensor comprises a CMOS image sensor.
23. A method of detecting analytes comprising contacting a fluid containing target analytes with an array as in any one of aspects 19-22.
24. A method of increasing the signal arising from analytes in an array formed on a CMOS image sensor comprising coupling a plurality of probes to a molecule selected from the group consisting of a polysaccharide, a hydrogel, and a branched oligonucleotide.
25. A method of detecting analytes in an array comprising increasing the frame integration time of a CMOS image sensor, thereby integrating analyte signal.
26. The method of aspect 25 further including the steps of storing integrated signal and repeating the integration at fixed time intervals.
27. A sensor device comprising,
   an optical digital image sensor;
   a low-light enclosure; and
   a conjugate probe array.
28. The sensor device of aspect 27 wherein the optical digital image sensor is a CMOS image sensor.
29. The sensor device of aspect 27 wherein the low-light enclosure further comprises a fluid entry opening having a septum.
30. A biosensor system comprising,
   a reading station having a first connector;
   a portable enclosure comprising;
   an optical digital image sensor;
   a low-light enclosure;
   a probe array;
   a second connector for attaching the portable enclosure to the first connector, thereby attaching the portable enclosure to the reading station.
31. The biosensor system of aspect 30 wherein the second connector is a circuit board edge connector.
32. The biosensor system of aspect 30 wherein the reading station comprises circuit means for hot swap of the portable enclosure.
33. The biosensor system of aspect 30 further comprising a means for cooling the optical digital image sensor.
34. An kit having component parts capable of being assembled into an assay system comprising a polysaccharide conjugate coupled to at least one recognition molecule and at least one signaling molecule.

## Claims

1. An array for detecting analytes with an image sensor, the array comprising:
- a complementary metal-oxide-semiconductor (CMOS) optical digital image sensor comprising as its top layer a transparent passivation layer; and
- a plurality of conjugate probes linked covalently to said transparent passivation layer;
wherein at least one conjugate of the plurality of conjugates comprises a target-binding probe moiety covalently coupled to a polymer.

2. An array according to claim 1, wherein the polymer is a branched polysaccharide selected from amylose, amylopectin, glycogen, dextran, cellulose, chitin, chitosan, peptidoglycan, glycosaminoglycan and mixtures thereof.

3. An array according to claim 2 wherein the branched polysaccharide is dextran.

4. An array according to any one of the preceding claims, wherein the target-binding probe moiety is an oligonucleotide, a peptide nucleic acid, a protein or an antibody or antibody fragment.

5. An array according to claim 4 wherein the target-binding probe moiety is an oligonucleotide.

6. An array according to any one of the preceding claims, wherein the target-binding probe moiety is coupled to the polymer via a linking group.

7. An array according to any one of claims 1 to 6, wherein the target-binding moiety was covalently coupled to the polymer via reaction with a photoreactive crosslinker or a heterobifunctional photoreactive cross-linker.

8. An array according to any one of claims 1 to 6, wherein the polymer is a polysaccharide, and wherein the target-binding moiety was covalently coupled to said polysaccharide via reaction with a carbonylating agent or an oxidizing agent.

9. A method of detecting analytes, the method comprising contacting a fluid containing target analytes with an array according to any one of the preceding claims.

## Patentansprüche

1. Array zum Erfassen von Analyten mit einem Bildsensor, wobei das Array umfasst:
- einen optischen digitalen Bildsensor auf Basis von komplementärer Metall-Oxid-Halbleiter(CMOS)-Technologie, umfassend als seine obere Schicht eine transparente Passivierungsschicht;
- eine Vielzahl von Konjugat-Sonden, welche kovalent mit der transparenten Passivierungsschicht verbunden sind; wobei wenigstens ein Konjugat aus der Vielzahl von Konjugaten eine Ziel-bindende Sonden-Moiety umfasst, welche mit einem Polymer gekoppelt ist.

2. Array nach Anspruch 1, wobei das Polymer ein verzweigtes Polysaccharid ist, welches ausgewählt ist aus Amylose, Amylopectin, Glykogen, Dextran, Zellulose, Chitin, Chitosan, Peptidoglycan, Glycosaminoglycan und Mischungen davon.

3. Array nach Anspruch 2, wobei das verzweigte Polysaccharid Dextran ist.

4. Array nach einem der vorhergehenden Ansprüche, wobei die Ziel-bindende Sonden-Moiety ein Oligonukleotid, eine Peptidnukleinsäure, ein Protein oder ein Antikörper oder Antikörperfragment ist.

5. Array nach Anspruch 4, wobei die Ziel-bindende Sonden-Moiety ein Oligonukleotid ist.

6. Array nach einem der vorhergehenden Ansprüche, wobei die Ziel-bindende Sonden-Moiety über eine Verbindungsgruppe mit dem Polymer gekoppelt ist.

7. Array nach einem der Ansprüche 1 bis 6, wobei die Ziel-bindende Moiety durch Reaktion mit einem photoreaktiven Kreuzvernetzer oder einem heterobifunktionalen photoreaktiven Kreuzvernetzer kovalent mit dem Polymer gekoppelt wurde.

8. Array nach einem der Ansprüche 1 bis 6, wobei das Polymer ein Polysacharrid ist, und wobei die Ziel-bindende Moiety durch Reaktion mit einem Carbonylisierungsmittel oder einem Oxidationsmittel kovalent mit dem Polysaccharid gekoppelt wurde.

9. Verfahren zum Erfassen von Analyten, wobei das Verfahren ein Inkontaktbringen eines Fluid enthaltenden Zielanalyts mit einem Array nach einem der vorhergehenden Ansprüche umfasst.

## Revendications

1. Réseau pour la détection d'analytes avec un capteur d'image, le réseau comprenant :
- un capteur d'image numérique optique métal-oxyde-semi-conducteur complémentaire (CMOS) comprenant, comme couche supérieure, une couche de passivation transparente ; et
- une pluralité de sondes conjuguées liées par liaison covalente à ladite couche de passivation transparente ;
dans lequel au moins un conjugué de la pluralité de conjugués comprend une fraction de sonde de liaison à la cible couplée par liaison covalente à un polymère.

2. Réseau selon la revendication 1, dans lequel le polymère est un polysaccharide ramifié choisi parmi l'amylose, l'amylopectine, le glycogène, le dextrane, la cellulose, la chitine, le chitosane, le peptidoglycane, le glycosaminoglycane et leurs mélanges.

3. Réseau selon la revendication 2, dans lequel le polysaccharide ramifié est le dextrane.

4. Réseau selon l'une quelconque des revendications précédentes, dans lequel la fraction de sonde de liaison à la cible est un oligonucléotide, un acide nucléique peptidique, une protéine ou un anticorps ou fragment d'anticorps.

5. Réseau selon la revendication 4, dans lequel la fraction de sonde de liaison à la cible est un oligonucléotide.

6. Réseau selon l'une quelconque des revendications précédentes, dans lequel la fraction de sonde de liaison à la cible est couplée au polymère par l'intermédiaire d'un groupe de liaison.

7. Réseau selon l'une quelconque des revendications 1 à 6, dans lequel la fraction de liaison à la cible a été couplée par liaison covalente au polymère par le biais d'une réaction avec un agent de réticulation photoréactif ou un agent de réticulation photoréactif hétérobifonctionnel.

8. Réseau selon l'une quelconque des revendications 1 à 6, dans lequel le polymère est un polysaccharide, et dans lequel la fraction de liaison à la cible a été couplée par liaison covalente audit polysaccharide par le biais d'une réaction avec un agent de carbonylation ou un agent oxydant.

9. Procédé de détection d'analytes, le procédé comprenant la mise en contact d'un fluide contenant des analytes cibles avec un réseau selon l'une quelconque des revendications précédentes.
